# EUROPEAN PATENT SPECIFICATION

(11) **EP 3 136 862 B1**
(45) Date of publication and mention of the grant of the patent: **25.03.2020**
(21) Application number: 15710303.7
(22) Date of filing: 10.02.2015
(51) Int. Cl.: A01N 59/00, A01N 37/16, A61K 8/22, A01P 1/00, A61L 2/20

(54) **PROCESS OF KILLING SPORES USING VAPOROUS PEROXIDE COMPOSITION**
VERFAHREN ZUM ABTÖTEN VON SPOREN MIT DAMPFFÖRMIGER PEROXIDZUSAMMENSETZUNG
PROCÉDÉ POUR TUER DES SPORES AVEC UNE COMPOSITION DE PEROXYDE VAPOREUX

(30) Priority: 28.04.2014 US 201414262840; 28.10.2014 US 201414525497; 11.11.2014 US 201414538011
(43) Date of publication of application: 08.03.2017
(73) Proprietor: American Sterilizer Company, Mentor, OH 44060 (US)
(72) Inventor: BURKE, Peter A., Concord, Ohio 44077 (US); LEGGETT, Mark James, Cardiff Wales CF23 6DT (GB); CENTANNI, Michael A., Parma, Ohio 44130 (US)
(74) Representative: Ström & Gulliksson AB
(86) International application number: PCT/US2015/015090
(87) International publication number: WO 2015/167643

(56) References cited:
- WO-A1-88/08667
- WO-A1-2008/111893
- US-A1- 2005 095 168
- US-A1- 2009 061 017
- US-A1- 2010 284 855
- US-A1- 2011 076 192
- US-A1- 2012 189 494
- US-A1- 2014 037 499

## Description

### Technical Field

This invention relates to a non-therapeutic decontamination or sterilization process wherein spores are killed using a sporicidal vaporous mixture derived from an aqueous composition containing hydrogen peroxide and peracetic acid.

### Background

Spores are a highly resistant, dormant cell type formed by some types of bacteria. Endospores (or simply spores) form within the vegetative mother cell in response to adverse changes in the environment, most commonly nutrient depletion. The mother cell undergoes an asymmetrical cell division, where it replicates its genetic material, which is then surrounded by multiple concentric and spore specific layers. The mother cell then disintegrates, releasing the mature dormant spore which requires neither nutrients, water nor air for survival and is protected against a variety of trauma, including extremes of temperature, radiation, and chemical assault. Spore forming bacteria cause a number of serious diseases in humans, including botulism, gas gangrene, tetanus, and acute food poisoning. Anthrax results from infection by the aerobic spore form *Bacillus anthracis.*

Further, in the art:
US 2012/189494 A1 discloses a method of sterilizing a surface comprising treating such surface with a vapor comprising peracetic acid at a concentration of at least about 3500 ppm at between about 57° and about 75° C.
US 2011/076192 A1 discloses a method of sterilizing an article by sequentially exposing the article to hydrogen peroxide and ozone is disclosed. The article is exposed under vacuum first to an evaporated aqueous solution of hydrogen peroxide and subsequently to an ozone containing gas.
WO 2008/111893 A1 discloses a method of producing a packaging container with low microbiological load by treating the packaging material with a combination of a chemical sterilization agent and UV-light.
US 2010/284855 A1 discloses a process, which combines the advantages of refrigeration in a synergistic manner with the advantages of hydrogen peroxide and other organic and inorganic peroxygen compounds.
US 2014/037499 A1 A system and process for disinfecting rooms such as health care facility rooms with an oxygen/ozone mixture is described, which is effective to combat "superbugs" such as *Clostridium difficile* (*C. difficile*); *E. coli*; *Pseudomonas aeruginosa*; methicillin-resistant *Staphylococcus aureus* (MRSA); and vancomycin-resistant *Enterococcus* (VRE).
US 2005/095168 A1 discloses vaporizer heating apparatus comprised of electromagnetically responsive material and electrically non-conductive material. A antimicrobial fluid to be vaporized, such as water or hydrogen peroxide solution, is supplied to the heating apparatus where it is converted to a vapor.
WO 88/08667 A1 discloses a stable, shippable microbicidal composition including between about 0.2 to 8 % hydrogen peroxide, about 0.2 to 11 % peracetic plus acetic acid, 0 to about 1.0 % sequestrant such as organic phosphonic acid or its salt and water, and surfactant between 0 and about 1 % with the ratio of total acid to H₂O₂ being between about 1.0 and 11.
US 2009/061017 A1 discloses shelf stable and/or less corrosive peroxycarboxylic acid antimicrobial compositions, including ready-to-use compositions. Shelf stable compositions can include defined ratios of hydrogen peroxide to peroxycarboxylic acid and/or hydrogen peroxide to protonated carboxylic acid, but need not include strong acid

### Summary

Spores are difficult to kill and a problem in the art of decontamination and sterilization relates to providing an effective process for killing spores. This invention provides a solution to this problem.

This invention relates to a non-therapeutic process for killing bacterial spores, comprising: forming a dispersion of droplets of an aqueous composition in a carrier gas, the aqueous composition comprising water and hydrogen peroxide, the aqueous composition having a concentration of peroxide which is hydrogen peroxide in the range from 0.1 to 6.5% by weight. The aqueous composition further comprises an antimicrobial agent which is peracetic acid, the concentration of the antimicrobial agent being in the range from 0.001 to 0.16% by weight, the weight ratio of the antimicrobial agent to the peroxide being in the range from 0.001 to 0.2, and wherein the carrier gas is selected from air, nitrogen, carbon dioxide, helium, argon, or a mixture of two or more thereof. The process further comprises vaporizing the droplets to form a sporicidal vaporous mixture; and contacting spores with the sporicidal vaporous mixture for a sufficient period of time to effect at least a 4 log reduction, or at least a 5 log reduction, or at least a 6 log reduction in the number of spores capable of returning to vegetative growth, the spores being in a defined area and the concentration of the peroxide in the defined area is in the range from 5 to 1000 parts per million.

In an embodiment, the weight ratio of antimicrobial agent which is peracetic acid to the peroxide which is hydrogen peroxide in the aqueous composition is in the range from 0.008 to 0.2, or from 0.01 to 0.1.

The time required to effect at least a 4 log reduction, or at least a 5 log reduction, or at least a 6 log reduction in the number of spores capable of returning to vegetative growth is in the range from 30 seconds to 20 minutes, or from 30 seconds to 10 minutes.

### Brief Description of the Drawings

Fig. 1 is a schematic illustration of a bacterial spore that can be killed in accordance with the invention.
Fig. 2 is a schematic illustration of a vaporization system that may be used in accordance with the invention.

### Detailed Description

All ranges and ratio limits disclosed in the specification and claims may be combined in any manner. It is to be understood that unless specifically stated otherwise, references to "a," "an," and/or "the" may include one or more than one, and that reference to an item in the singular may also include the item in the plural.

The phrase "and/or" should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

The phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

The transitional words or phrases, such as "comprising," "including," "carrying," "having," "containing," "involving," "holding," and the like, are to be understood to be open-ended, i.e., to mean including but not limited to.

The term "killing" (or "kill") spores refers to rendering the spores incapable of returning to vegetative growth. In an embodiment, the term killing spores refers to rendering the spores incapable of reproduction, metabolism and/or growth.

The term "log reduction" is a mathematical term to show the number of live spores killed by contacting the spores with the aqueous composition of the invention. A "4 log reduction" means that the number of live spores is 10,000 times smaller. A "5 log reduction" means that the number of live spores is 100,000 times smaller. A "6 log reduction" means that the number of live spores is 1,000,000 times smaller.

The term "antimicrobial agent" refers to a substance that kills microorganisms or inhibits their growth.

The term "disinfectant" refers to a substance that is applied to non-living objects to kill or inhibit the growth of microorganisms that are on the objects.

The term "antibiotic" refers to a substance that kills or inhibits the growth of microorganisms within the body.

The term "antiseptic" refers to a substance that kills or inhibits the growth of microorganisms on living tissue.

The term "biocide" refers to a substance that kills or inhibits the growth of living organisms. The biocide can be a pesticide. The biocide can be a fungicide, herbicide, insecticide, algaecide, molluscicide, miticide or rodenticide.

The term "sanitizer" refers to a substance that cleans and disinfects.

Sterilization with respect to spores is often taken as referring to a process for achieving a total absence of living spores. Processes that are less rigorous than sterilization may include decontamination processes. The process provided for herein is used to achieve at least a 4 log reduction, or at least a 5 log reduction, or at least a 6 log reduction in the number of spores capable of returning to vegetative growth, or in an embodiment capable of reproduction, metabolism and/or growth. When at least a 6 log reduction is achieved, the process may be referred to as a sterilization process. When a 4 log reduction or a 5 log reduction is achieved, the process may be considered to be less rigorous than a sterilization, but nevertheless useful for various decontamination applications.

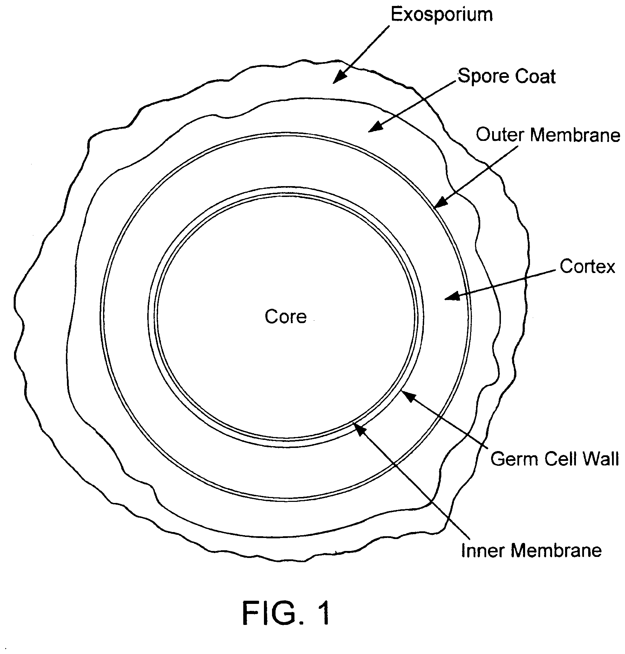

Bacterial spores typically comprise multiple concentric layers surrounding a central core. This is illustrated in Fig. 1 wherein a bacterial spore is shown which has a central core, inner membrane, germ cell wall, cortex, outer membrane, spore coat and occasionally an exosporium. Oxidizing agents for years have been thought to attack DNA, RNA, protein and most organic matter equally. However, while not wishing to be bound by theory, with the present invention it is believed that the mechanism that is provided involves the peroxide (e.g., hydrogen peroxide) first piercing holes in multiple layers surrounding the central core of the spores, and then the antimicrobial agent advancing through the pierced holes and attacking the central core to kill the spores. This mechanism is believed to occur when using sporicidal vaporous mixtures with relatively low concentrations of peroxide. The antimicrobial agent is also present at a relatively low concentration. This mechanism is believed to occur when relatively low concentrations of the antimicrobial agent and peroxide are used, as indicated above, and the antimicrobial agent to peroxide weight ratio is relatively low.

Advantages of the inventive process include relatively low costs due to the fact that the concentrations of the antimicrobial agent and peroxide used in the process are relatively low in comparison to concentrations used in other processes using these ingredients. Other advantages include low levels of corrosion of surfaces treated due to the low concentrations of the antimicrobial agent and peroxide.

The water may comprise tap water, deionized water, distilled water, water purified by osmosis, or a mixture of two or more thereof.

Peroxides are defined as compounds containing an oxygen-oxygen single bond, or a peroxide group or peroxide ion. In the present invention, the peroxide is hydrogen peroxide.

The hydrogen peroxide may be derived from any source of hydrogen peroxide. Hydrogen peroxide is typically available as a solution in water. Hydrogen peroxide concentrations of about 3 to about 8% by weight may be used. Commercial grades of about 30% to about 40% by weight, or about 35% by weight, hydrogen peroxide may be used. Commercial grades of about 70 to about 98% by weight hydrogen peroxide may be used. The higher concentrations would be diluted to provide the desired concentrations of hydrogen peroxide that are indicated above.

The antimicrobial agent in the inventive process is peracetic acid.

The carrier gas is selected from air, nitrogen, carbon dioxide, helium, argon, or a mixture of two or more thereof. In an embodiment, the carrier gas comprises air. In an embodiment, the carrier gas comprises a gaseous antimicrobial agent.

The gaseous antimicrobial agent may comprise ozone, chlorine dioxide, nitric oxide, or a mixture of two or more thereof.

The inventive process comprises forming droplets of the aqueous composition, vaporizing the droplets to form the sporicidal vaporous mixture, and contacting spores with the sporicidal vaporous mixture for a sufficient period of time to effect a desired level of reduction of at least a 4 log reduction, or at least a 5 log reduction, or at least a 6 log reduction in the number of spores capable of returning to vegetative growth, or in an embodiment, capable of reproduction, metabolism and/or growth. The droplets are dispersed in the carrier gas.

The spores to be killed are bacterial spores. The spores may comprise bacteria of the *Bacillus* or *Clostridia* genera. The spores may comprise *Geobacillus stearothermophilus, Bacillus atrophaeus, Bacillus subtilis, Bacillus pumilus, Bacillus coagulans, Clostridium sporogenes, Bacillus subtilis globigii, Bacillus cereus, Bacillus circulans, Bacillus anthracis,* or a mixture of two or more thereof. The spores may comprise one or more *Bacillus subtilis* strains and/or wild type *Bacillus subtilis* spores.

The temperature of the sporicidal vaporous mixture when contacting the spores may be in the range from about 10°C to about 70°C, or from about 20°C to about 60°C, or from about 25°C to about 55°C, or from about 30°C to about 50°C. The temperature may be in the range from about 20°C to about 26°C, or from about 21 °C to about 25°C, or from about 22°C to about 24°C, or about 22°C, or about 23°C. The temperature may be room temperature.

The sporicidal vaporous mixture is used in a defined area (e.g., decontamination or sterilization chamber, room, building, etc.) wherein the pressure is atmospheric pressure, above atmospheric pressure or below atmospheric pressure.

The spores that may be treated (i.e., killed) are in the defined area. The defined area may comprise a decontamination chamber. The defined area may comprise a sterilization chamber. The defined area may comprise a large enclosure such as a building or a room. The defined area may comprise a hospital, laboratory, office building, airplane, airport hanger, railroad car, bus, truck, automobile, hotel, or manufacturing facility.

The spores to be killed may be on a substrate. The substrate may be made of a material comprising metal, rubber, plastic, glass, wood, painted or coated surface, or a combination of two or more thereof. The substrate may comprise a table top, counter top, floor, wall, ceiling, window, door, door handle, sink, faucet, toilet or toilet seat. The substrate may comprise a medical, dental, pharmaceutical, veterinary or mortuary device. The substrate may comprise an endoscope. The substrate may comprise a pharmaceutical, food or beverage packaging item. The substrate may comprise processing equipment for pharmaceuticals or food. The substrate may comprise a freeze dryer or meat processing equipment.

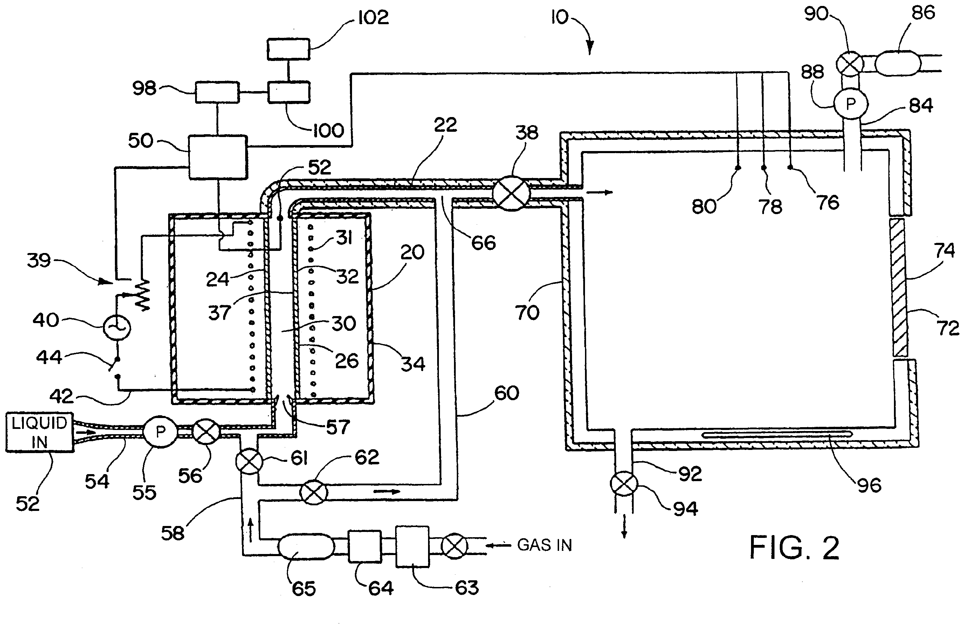

A system 10 for killing spores, which can be used in accordance with the invention, is illustrated in Fig. 2. Referring to Fig. 2, the system 10 includes flash vaporizer 20 and chamber 70. Items contaminated with spores are loaded into the chamber 70 through opening 72. Door 74 is then closed to seal the chamber 70. The sporicidal vaporous mixture is formed in vaporizer 20, flows from vaporizer 20 through tubular line 22 into chamber 70 where it contacts the spores and the contaminated items and kills the spores.

The vaporizer 20 includes an induction vessel 24, which is positioned in a magnetic field and is heated by electric current inductively generated in the induction vessel 24 by the magnetic field. The induction vessel 24 transfers heat to heating tube 26 via conduction, radiation and/or convection.

The heating tube 26 has a tube wall 28 defining an interior passage or bore 30, which may be cylindrical in shape. The heating tube 26 may be formed from an electrically and thermally conductive material, such as iron, carbon steel, stainless steel, aluminum, copper, brass, bronze, electrically conductive ceramic and polymer composites, or other materials capable of being inductively heated. An induction coil 31 is wrapped around an outer surface 32 of the tube 26 in a helix, along all or a portion of the tube length. The induction coil 31 may be spaced from the tube by a layer of insulation material (not shown in the drawing). An electrically insulative housing 34 surrounds the coil 31 and insulation material.

The aqueous composition flows from liquid container 52 through tubular line 54 towards heating tube 26. The carrier gas flows from carrier gas line 58 into tubular line 54 where it contacts the aqueous composition. In the tubular line 54, the carrier gas disperses the aqueous composition to form a dispersion of droplets of the aqueous composition in the carrier gas. The heating tube 26 provides bore 30 for receiving the dispersion and vaporizing the droplets. The heating tube 26 may be sized to receive a volume of the dispersion that is sufficiently small so that the droplets can be vaporized rapidly as they enter and contact the interior walls of the heating tube 26 in a flash vaporization process. While the heating tube 26 is shown in Fig. 2 as being vertically aligned along its axis, it is to be appreciated that the heating tube 26 may alternatively be aligned horizontally or have portions arranged with different orientations. Also, although not shown in Fig. 2, the interior heating tube 26 may include interior baffles which extend from the interior walls of the heating tube 26 into the bore 30. These baffles may be used to provide turbulence to the flow of the dispersion, and enhance the transfer of heat to the droplets.

An upper end or outlet 36 of the heating tube 26 is fluidly connected with the tubular line 22. Valve 38 in the tubular line 22 may be used to adjust the amount of the sporicidal vaporous mixture flowing into the chamber 70. The tubular line 22 or a fitting (not shown) connecting the tubular line 22 with the heating tube 26, may be formed of a material such as copper, brass or polymer.

An AC source 40 supplies an alternating current to the coil 31 via electric circuit 42. In response to the applied current, the coil 31 produces an alternating magnetic field, which passes through the heating tube 26. This may cause eddy currents which heat the tube 26. The heat passes through to an inner surface 37 of the tube 26 and heats the dispersion flowing through the bore 30. The electrical current and the rate of heating of the heating tube 26 may be adjustable, for example, with a current adjustment mechanism 39. The current adjustment mechanism 39, which may comprise a pulse width modulator, a variable resistor, or the like, in the electrical circuit 42 connecting the AC source 40 and the induction coil 31. Alternatively, or additionally, the current adjustment mechanism 39 may include an on/off switch 44 for the circuit 42.

The current adjustment mechanism 39 may be controlled by controller 50, which may also control other aspects of the system 10. For example, the controller 50 may receive temperature measurements from temperature monitor 52, which may be in the form of a thermocouple, and is positioned adjacent the outlet end 36 of the heating tube 26, or elsewhere in the system 10, such as in the tubular line 22. The controller 50 may control the current adjustment mechanism 39 in response to the measured temperature in the chamber 70 to maintain a preselected operating temperature. The controller 50 may also be connected with one or more of temperature monitors 76 and pressure monitors 78 positioned within the chamber 70 or elsewhere in the system 10. The controller 50 may be used to regulate the vaporizer 20 to maintain the desired temperature and pressure.

The flow of the aqueous composition into inlet tubular line 54 may be regulated by adjustable inlet valve 56. The valve 56 may be a solenoid valve controlled by controller 50. The inlet tubular line 54 is connected to a second end or inlet end 57 of the heating tube 26. As with the outlet tubular line 22, the inlet tubular line 54, or a fitting (not shown) connecting the inlet tubular line 54 with the heating tube 26, may be formed from copper, brass or polymer.

The inductively generated heat flash vaporizes the droplets in the bore 30 to produce the sporicidal vaporous mixture. The dispersion may be introduced to the bore 30 as a continuous stream under pressure.

The droplets may be vaporized when they contact the inner surface 37 of the heating tube 26. The carrier gas may be used to assist in propelling the droplets through the bore 30. Additionally, carrier gas line 60 may be connected with the tubular line 22, such that additional carrier gas may be mixed in mixing zone 66 with the already formed sporicidal vaporous mixture. Mixing the carrier gas with the sporicidal vaporous mixture after vapor formation in the heating tube 26 may increase the throughput of the heating tube 26. Valves 61 and 62 in the carrier gas lines 58 and 60 may be used to regulate the flow rate of carrier gas through the lines 58 and 60, respectively.

The carrier gas may be air at atmospheric pressure or it may be supplied from a tank or other reservoir (not shown). The incoming carrier gas may pass through filter 63, which may be a high efficiency particulate air (HEPA) filter, to remove airborne particulates, through a dryer 64 to remove excess moisture, and heated by a heater 65 to raise the temperature of the carrier gas.

The pressure of the carrier gas supplied to lines 58 and 60 may vary with the production rate of the sporicidal vaporous mixture and restrictiveness of passages in the vaporizer 20, and typically may vary from about 1 to about 2 atmospheres absolute (101.3 to 202.7 kilopascals absolute), i.e., about 0 to about 1 atmosphere gauge (0 to 101.3 kilopascals), or about 0.06 to about 0.14 atmosphere gauge (6.1 to 14.2 kilopascals).

The flash vaporization and sweeping carrier gas may ensure that the sporicidal vaporous mixture does not condense and form a puddle in the heating tube 26.

The carrier gas may tend to cool the bore 30, reducing the rate at which the droplets may be vaporized. Consequently, it may be desirable to maintain the carrier gas at or slightly above a minimum flow rate needed to carry the dispersion through the bore 30 without significant degradation of the vapor, but at a flow rate which is low enough such that appreciable cooling of the dispersion by the carrier gas does not occur. The flow rate of the part of the carrier gas that flows through line 58 and bore 30 may be lower than the flow rate of the part of the carrier gas that flows through line 60. The part of the carrier gas that flows through line 60 may be injected into the sporicidal gaseous mixture flowing out of the bore 30 in mixing zone 66, which is positioned downstream of the bore 30. The resulting sporicidal vaporous mixture may then flow into the chamber 70 via line 22.

A sensor 80, such as a peroxide sensor, may be used to detect the concentration of peroxide in the chamber 70. The controller 50 may receive the detected concentration measurements or signals indicative thereof, as well as temperature and pressure measurements from monitors 76 and 78, and regulate the supply of fresh sporidical gaseous mixture to the chamber 70. Alternatively, the controller 50 may be preprogrammed with expected concentrations of peroxide or other data which allows the controller 50 to maintain selected chamber conditions by controlling and/or measuring various parameters of the system, such as chamber temperature and pressure, as well as peroxide and carrier gas flow rates.

Spent gas and vapor may exit the chamber 70 via outlet line 84. The spent gas and vapor may pass through a destroyer 86, which may be a catalytic converter, to convert any remaining peroxide to oxygen and water, before releasing it to the atmosphere. The removal of spent gas and vapor may be assisted using vacuum pump 88 and valve 90. Spent liquid may be removed from the chamber 70 through line 92 using valve 94.

Alternatively, the outlet line 84 may be coupled with the carrier gas inlet line 58 and/or 60 as a recirculating flow through system, whereby the spent gas and vapor, preferably after passing through the destroyer 86, is returned to the inlet line 58, intermediate the filter 63 and dryer 64, or prior to the filter 63, such that the spent gas and vapor is dried and heated before mixing once more with the aqueous composition, droplets or vapor.

The temperature within the chamber 70 may be at room temperature, below room temperature, or above room temperature. The temperature may be in the range from about 10°C to about 70°C, or from about 20°C to about 60°C, or from about 25°C to about 55°C, or from about 30°C to about 50°C. The temperature may be in the range from about 20°C to about 26°C, or from about 21 °C to about 25°C, or from about 22°C to about 24°C, or about 22°C, or about 23°C. If it is desired to heat the chamber 70, a heater 96, which may be a resistance heater which surrounds all or part of the chamber 70 may be used. The heater 96 may be controlled using the controller 50. The pressure within the chamber 70 may be atmospheric pressure, below atmospheric pressure, or above atmospheric pressure.

The sporicidal vaporous mixture within the chamber 70 may be continually or intermittently replenished. The system 10 may be operated as a deep vacuum system, in which the chamber 70 is evacuated to a pressure of, for example about 10 torr or below, prior to introduction of the sporicidal vaporous mixture. One or more pulses of the sporicidal vaporous mixture may be introduced to the chamber 70, with vacuum pulses between them.

In an embodiment, the spores may be positioned in a lumen, and a vacuum may be used to draw the sporicidal vaporous mixture into the lumen in contact with the spores. This is disclosed in U.S. Patent 5,527,508.

For sterilizing larger enclosures, such as buildings, rooms, and the like, additional vaporizers 20 may be employed, each one being under the control of the controller 50.

The progress of the decontamination or sterilization process may be monitored using one or more decontamination or sterilization indicators. These indicators may contain a biological indicator. The biological indicator may comprise one or more test organisms which may be more resistant to the decontamination or sterilization process than the organisms to be destroyed by the decontamination or sterilization process. The test organism may be placed in contact with an incubation medium to determine whether the decontamination or sterilization process was effective.

### Reference examples

The efficacy of the potentiation of activity is assessed using a time kill suspension test method and spores of *Bacillus subtilis.*

Peracetic acid (PAA) and hydrogen peroxide (H₂O₂) are prepared as concentrated stocks (3x concentrate). Each test contains 100 µl of the PAA concentrate and 100 µl of the H₂O₂ concentrate. Controls containing only PAA or H₂O₂ are also prepared. These contain 100 µl of either the PAA concentrate or H₂O₂ concentrate and 100 µl of de-ionized water. To each test, 100 µl of spores are added while starting the timer concurrently. The samples are mixed thoroughly. The temperature of the samples is room temperature (i.e., about 26°C). At the appropriate contact times, 10 µl of the appropriate test sample are placed into 90 µl of the appropriate neutralizing solution, mixed thoroughly and incubated for at least 10 minutes. Ten fold serial dilutions are prepared through 10⁻⁶ and plated using the drop counting method. The plates are then incubated aerobically at 37 °C for 1-2 days. Following incubation, colony forming units (CFU) are counted using standard plate count techniques and converted to log10 values for analysis.

The results are indicated in the tables below.

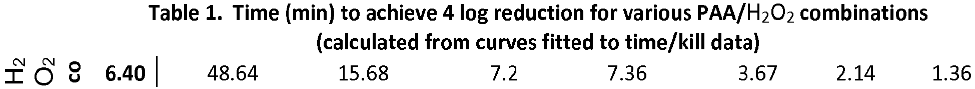

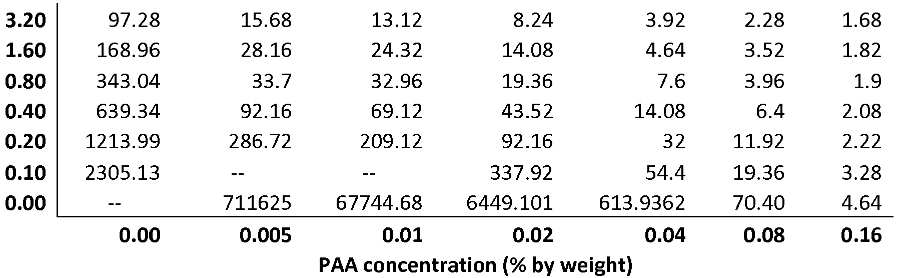

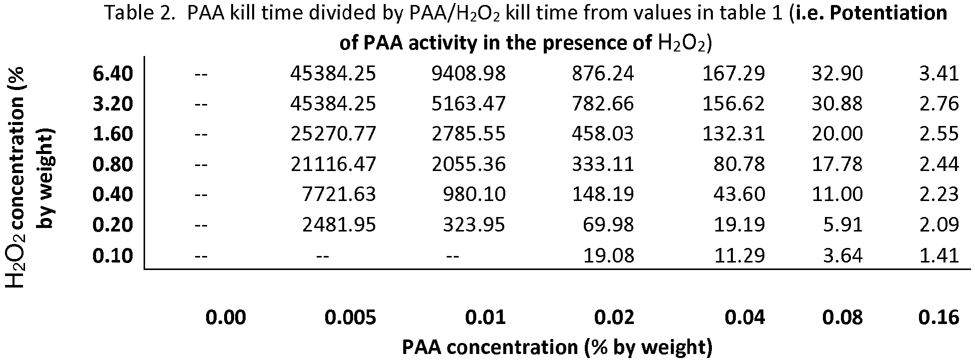

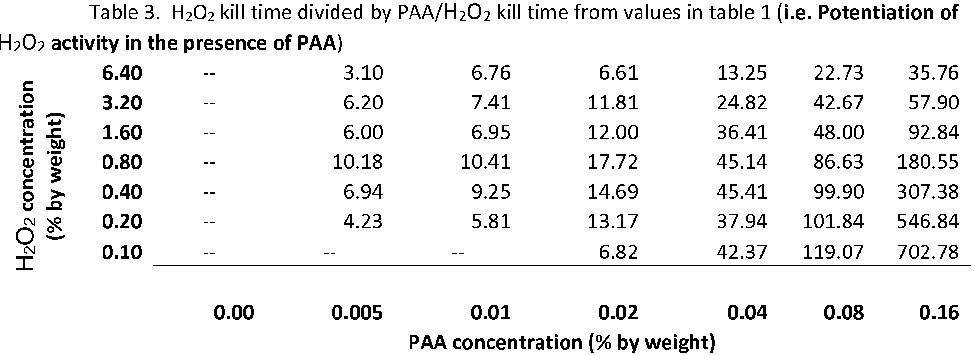

The values shown in Table 1 represent the time taken (minutes) to achieve a 4 log reduction in spore count in the presence of either PAA or H₂O₂ alone, or in combination with each other. For PAA concentrations 0.005, 0.01, 0.02 and 0.04% (in the absence of H₂O₂), the values shown are extrapolated based on the experimental data obtained for PAA concentrations 0.08, 0.16 and 0.32%. Similarly, for H₂O₂ concentrations 0.1, 0.2 and 0.4% (in the absence of PAA), the values shown are extrapolated from experimental data. All other values are generated from spore kill data.

Table 2 illustrates the potentiation of spore killing by PAA when in the presence of H₂O₂. At higher PAA concentrations (0.08 and 0.16% PAA) relatively little activity is gained by the addition of even very high concentrations of H₂O₂. For example, 0.16% PAA is only 3.41 times more active in the presence of 6.4% H₂O₂, as compared to the activity of 0.16% PAA alone.

However, as the concentration of PAA is reduced, the effect of adding H₂O₂ becomes more dramatic, with PAA spore killing activity being hundreds, thousands and even tens of thousands of times greater when in the presence of low concentrations of H₂O₂. For example, 0.02% PAA is 333.11 times more active in combination with 0.8% H₂O₂ than when used alone.

Table 3 illustrates the potentiation of spore killing by H₂O₂ when in the presence of PAA. The enhancement of the spore killing activity of H₂O₂ when in the presence of PAA is far less pronounced, with relative improvement in the spore killing activity of H₂O₂ in combination with all but the highest concentrations of PAA being no greater than about 100 times.

## Claims

1. A non-therapeutic process for killing bacterial spores, comprising:
forming a dispersion of droplets of an aqueous composition in a carrier gas, the aqueous composition comprising water and peroxide, the aqueous composition having a concentration of peroxide which is hydrogen peroxide in the range from 0.1 to 6.5% by weight, wherein the aqueous composition further comprises an antimicrobial agent which is peracetic acid, the concentration of the antimicrobial agent being in the range from 0.001 to 0.16% by weight, the weight ratio of the antimicrobial agent to the peroxide peroxide being in the range from 0.001 to 0.2, and wherein the carrier gas is selected from air, nitrogen, carbon dioxide, helium, argon, or a mixture of two or more thereof;
vaporizing the droplets to form a sporicidal vaporous mixture; and
contacting the bacterial spores with the sporicidal vaporous mixture for a sufficient period of time to effect at least a 4 log reduction in the number of spores capable of returning to vegetative growth, the spores being in a defined area, the concentration of the peroxide in the defined area being in the range from 5 to 1000 parts per million.

2. The process of claim 1 wherein the carrier gas is air.

3. The process of claim 1 wherein the sporicidal vaporous mixture contacts the spores for a sufficient period of time to effect at least a 6 log reduction in the number of spores capable of returning to vegetative growth.

4. The process of claim 1 wherein the defined area comprises a decontamination chamber.

5. The process of claim 1 wherein the defined area comprises a sterilization chamber.

6. The process of claim 1 wherein the defined area comprises a building or a room.

7. The process of claim 1 wherein the spores are on a substrate.

8. The process of claim 7 wherein the substrate comprises a medical, dental, pharmaceutical, veterinary or mortuary device.

9. The process of claim 7 wherein the substrate comprises a pharmaceutical, food or beverage packaging item.

10. The process of claim 7 wherein the substrate comprises processing equipment for pharmaceuticals or food.

11. The process of claim 7 wherein the substrate comprises a freeze dryer or meat processing equipment.

12. The process of claim 1 wherein the temperature of the sporicidal vaporous mixture is in the range from 10°C to 70°C.

13. The process of claim 1 wherein the spores comprise bacteria of the *Bacillus* or *Clostridia* genera.

14. The process of claim 1 wherein the spores comprise *Geobacillus stearothermophilus, Bacillus atrophaeus, Bacillus subtilis, Bacillus pumilus, Bacillus coagulans, Clostridium sporogenes, Bacillus subtilis globigii, Bacillus cereus, Bacillus circulans, Bacillus anthracis,* or a mixture of two or more thereof.

15. The process of claim 1 wherein the spores comprise one or more *Bacillus subtilis* strains.

## Patentansprüche

1. Nicht-therapeutisches Verfahren zum Abtöten von bakteriellen Sporen, das Folgendes umfasst:
Bilden einer Dispersion von Tröpfchen einer wässrigen Zusammensetzung in einem Trägergas, wobei die wässrigen Zusammensetzung Wasser und Peroxid aufweist, wobei die wässrige Zusammensetzung eine Konzentration von Peroxid, das Wasserstoffperoxid ist, aufweist, die in dem Bereich von 0,1 bis 6,5 Gew.-% liegt, wobei die wässrige Zusammensetzung ferner ein antimikrobielles Agens aufweist, das Peressigsäure ist, wobei die Konzentration des antimikrobiellen Agens in dem Bereich von 0,001 bis 0,16 Gew.-% liegt, wobei das Gewichtsverhältnis des antimikrobiellen Agens zu dem Peroxid in dem Bereich von 0,001 bis 0,2 liegt, und wobei das Trägergas ausgewählt ist aus Luft, Stickstoff, Kohlendioxid, Helium, Argon oder einer Mischung aus zwei oder mehreren davon;
Verdampfen der Tröpfchen, um eine sporizide dampfförmige Mischung zu bilden; und
Kontaktieren der bakteriellen Sporen mit der sporiziden dampfförmigen Mischung für eine ausreichende Zeitdauer, um eine Reduzierung von zumindest 4 Log in der Anzahl von solchen Sporen zu bewirken, die in der Lage sind, zu vegetativem Wachstum zurückzukehren, wobei die Sporen in einem definierten Gebiet vorliegen, wobei die Konzentration des Peroxids in dem definierten Gebiet in dem Bereich von 5 bis 1000 Teilen pro Millionen liegt.

2. Verfahren nach Anspruch 1, wobei das Trägergas Luft ist.

3. Verfahren nach Anspruch 1, wobei die sporizide dampfförmige Mischung die Sporen für eine Zeitdauer kontaktiert, die ausreichend ist, um eine Reduzierung von zumindest 6 Log in der Anzahl von solchen Sporen zu bewirken, die in der Lage sind, zu vegetativem Wachstum zurückzukehren.

4. Verfahren nach Anspruch 1, wobei das definierte Gebiet eine Dekontaminationskammer umfasst.

5. Verfahren nach Anspruch 1, wobei das definierte Gebiet eine Sterilisationskammer umfasst.

6. Verfahren nach Anspruch 1, wobei das definierte Gebiet ein Gebäude oder einen Raum umfasst.

7. Verfahren nach Anspruch 1, wobei die Sporen auf einem Substrat vorliegen.

8. Verfahren nach Anspruch 7, wobei das Substrat eine medizinische, zahnmedizinische, pharmazeutische, veterinärmedizinische oder Leichenvorrichtung umfasst.

9. Verfahren nach Anspruch 7, wobei das Substrat einen Verpackungsgegenstand für Pharmazeutika, Lebensmittel oder Getränke umfasst.

10. Verfahren nach Anspruch 7, wobei das Substrat eine Verarbeitungsausstattung für Pharmazeutika oder Lebensmittel umfasst.

11. Verfahren nach Anspruch 7, wobei das Substrat einen Gefriertrockner oder eine Ausstattung zur Verarbeitung von Fleisch umfasst.

12. Verfahren nach Anspruch 1, wobei die Temperatur der sporiziden dampfförmigen Mischung in dem Bereich von 10°C bis 70°C liegt.

13. Verfahren nach Anspruch 1, wobei die Sporen Bakterien der Gattungen *Bacillus* oder *Clostridia* aufweisen.

14. Verfahren nach Anspruch 1, wobei die Sporen *Geobacillus stearothermophilus, Bacillus atrophaeus, Bacillus subtilis, Bacillus pumilus, Bacillus coagulans, Clostridium sporogenes, Bacillus subtilis globigii, Bacillus cereus, Bacillus circulans, Bacillus anthracis* oder eine Mischung von zwei oder mehreren davon aufweisen.

15. Verfahren nach Anspruch 1, wobei die Sporen einen oder mehrere Stämme von *Bacillus subtilis* aufweisen.

## Revendications

1. Procédé non thérapeutique pour tuer des spores bactériennes, comprenant :
la formation d'une dispersion de gouttelettes d'une composition aqueuse dans un gaz vecteur, la composition aqueuse comprenant de l'eau et du peroxyde, la composition aqueuse ayant une concentration de peroxyde qui est du peroxyde d'hydrogène dans la plage de 0,1 à 6,5 % en poids, dans lequel la composition aqueuse comprend en outre un agent antimicrobien qui est l'acide péracétique, la concentration de l'agent antimicrobien étant dans la plage de 0,001 à 0,16 % en poids, le rapport en poids entre l'agent antimicrobien et le peroxyde étant dans la plage de 0,001 à 0,2, et dans lequel le gaz vecteur est choisi parmi l'air, l'azote, le dioxyde de carbone, l'hélium, l'argon, ou un mélange de deux ou plus de ceux-ci ;
la vaporisation des gouttelettes pour former un mélange vaporeux sporicide ; et
la mise en contact des spores bactériennes avec le mélange vaporeux sporicide pendant une période de temps suffisante pour effectuer au moins une réduction de 4 log du nombre de spores capables de retourner à une croissance végétative, les spores étant dans une zone définie, la concentration du peroxyde dans la zone définie étant dans la plage de 5 à 1000 parties par million.

2. Procédé selon la revendication 1, dans lequel le gaz vecteur est l'air.

3. Procédé selon la revendication 1, dans lequel le mélange vaporeux sporicide entre en contact avec les spores pendant une période de temps suffisante pour effectuer au moins une réduction de 6 log du nombre de spores capables de retourner à une croissance végétative.

4. Procédé selon la revendication 1, dans lequel la zone définie comprend une chambre de décontamination.

5. Procédé selon la revendication 1, dans lequel la zone définie comprend une chambre de stérilisation.

6. Procédé selon la revendication 1, dans lequel la zone définie comprend un bâtiment ou une pièce.

7. Procédé selon la revendication 1, dans lequel les spores sont sur un substrat.

8. Procédé selon la revendication 7, dans lequel le substrat comprend un dispositif médical, dentaire, pharmaceutique, vétérinaire ou mortuaire.

9. Procédé selon la revendication 7, dans lequel le substrat comprend un objet d'emballage de produit pharmaceutique, d'aliment ou de boisson.

10. Procédé selon la revendication 7, dans lequel le substrat comprend un équipement de transformation de produits pharmaceutiques ou d'aliments.

11. Procédé selon la revendication 7, dans lequel le substrat comprend un lyophilisateur ou un équipement de transformation de viande.

12. Procédé selon la revendication 1, dans lequel la température du mélange vaporeux sporicide est dans la plage de 10 °C à 70 °C.

13. Procédé selon la revendication 1, dans lequel les spores comprennent des bactéries des genres *Bacillus* ou *Clostridia.*

14. Procédé selon la revendication 1, dans lequel les spores comprennent *Geobacillus stearothermophilus, Bacillus atrophaeus, Bacillus subtilis, Bacillus pumilus, Bacillus coagulans, Clostridium sporogenes, Bacillus subtilis globigii, Bacillus cereus, Bacillus circulans, Bacillus anthracis,* ou un mélange de deux ou plusieurs de ceux-ci.

15. Procédé selon la revendication 1, dans lequel les spores comprennent une ou plusieurs souches de *Bacillus subtilis.*
